(19) Europäisches Patentamt
European Patent Office
Office européen des brevets

(11) Veröffentlichungsnummer: **0 009 140**
**B1**

(12) # EUROPÄISCHE PATENTSCHRIFT

(45) Veröffentlichungstag der Patentschrift: **14.10.81**

(21) Anmeldenummer: **79103148.7**

(22) Anmeldetag: **27.08.79**

(51) Int. Cl.³: **C 07 D 211/46,** C 07 D 221/20, C 07 D 471/10 // (C07D471/10, 221/00, 221/00)

(54) **Polyalkylpiperidindiole und Verfahren zu ihrer Herstellung.**

(30) Priorität: **02.09.78 DE 2838364**

(43) Veröffentlichungstag der Anmeldung: **02.04.80 Patentblatt 80/7**

(45) Bekanntmachung des Hinweises auf die Patenterteilung: **14.10.81 Patentblatt 81/41**

(84) Benannte Vertragsstaaten: **CH DE FR GB IT NL**

(56) Entgegenhaltungen: **DE-A-2 126 954** **DE-A-2 204 659**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Wiezer, Hartmut, Dr., Hans-Fischer-Strasse 6, D-8906 Gersthofen (DE)**

EP 0 009 140 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Polyalkylpiperidindiole und Verfahren zu ihrer Herstellung

Die Erfindung betrifft neue Polyalkylpiperidindiole und ein Verfahren zu ihrer Herstellung. Die neuen Verbindungen entsprechen der allgemeinen Formel I

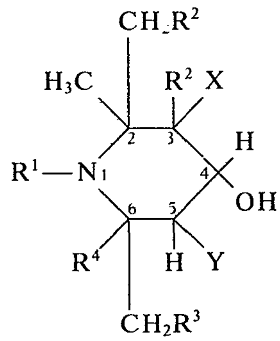

(I)

in welcher die Substituenten

X und Y verschieden sind und die Bedeutung einer Hydroxylgruppe oder eines Wasserstoffatoms haben,

$R^1$ Wasserstoff, Sauerstoff oder $C_1$- bis $C_{12}$-Alkyl, vorzugsweise Wasserstoff oder $C_1$- bis $C_4$-Alkyl und insbesondere Wasserstoff ist,

$R^2$ und $R^3$ entweder gleich sind und Wasserstoff oder eine $C_1$- bis $C_5$-Alkylgruppe und insbesondere Wasserstoff bedeuten, wobei dann

$R^4$ eine Methylgruppe ist, oder auch

$R^2$ Wasserstoff oder $C_1$- bis $C_5$-Alkyl ist und

$R^3$ und $R^4$ zusammen mit den Kohlenstoffatomen, an die sie gebunden sind, eine $C_5$- oder $C_6$-Cycloalkylgruppe oder eine Gruppe der Formel

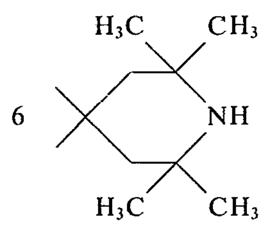

darstellen.

In den neuen Verbindungen besitzt das Ringstickstoffatom basische Eigenschaften, wenn es durch Wasserstoff oder Alkyl substituiert ist. In diesen Fällen können die Verbindungen auch in Form von Salzen mit anorganischen oder organischen Säuren vorliegen. Beispiele für solche Salze sind Phosphate, Sulfate, Chloride, Acetate, Laurate, Stearate, Succinate, Sebacate, Maleate, Citrate, Tartrate, Oxalate, Benzoate, Sulfonate, Phosphonate usw.

Von den neuen Polyalkylpiperidindiolen seien im einzelnen beispielsweise genannt:

2,2,6,6-Tetramethylpiperidin-3,4-diol
1,2,2,6,6-Pentamethylpiperidin-3,4-diol
1,9-Diaza-2,2,8,8,10,10-hexamethyl-spiro-[5,5]-undecan-3,4-diol
1,9-Diaza-2,2,8,8,10,10-hexamethyl-spiro[5,5]-undecan-4,5-diol
2,3,6-Trimethyl-2,6-diäthylpiperidin-3,4-diol
2,3,6-Trimethyl-2,6-diäthylpiperidin-4,5-diol
2,2,6,6-Tetramethylpiperidin-3,4-diolhydrochlorid

Die neuen Verbindungen werden gemäß dem folgenden Reaktionsschema, in dem die Substituenten $R^1$ bis $R^4$ die weiter vorne angegebene Bedeutung haben, erhalten, indem man Polyalkylpiperideine der allgemeinen Formeln (IIIa) und (IIIb) mit Persäuren zu Epoxiden umsetzt und an dieselben Wasser anlagert. Die Polyalkylpiperideine ihrerseits sind aus Polyalkylpiperidin-4-olen (II) durch Wasserabspaltung nach literaturbekannten Verfahren bzw. in Anlehnung an diese zugänglich (z. B. E. Fischer, Ber. 16, S. 1604; Ber. 17, S. 1790).

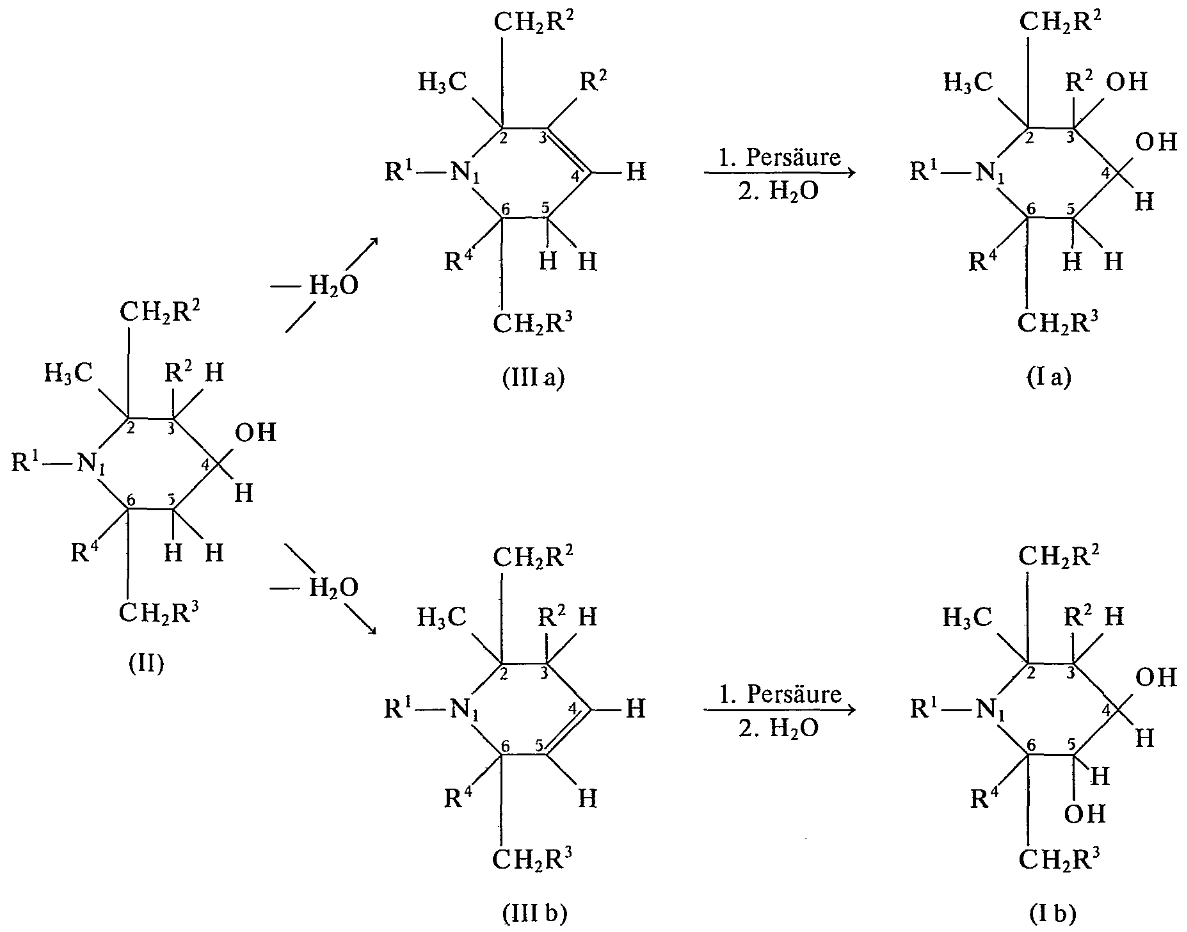


(II) (III a) (I a) (III b) (I b)

Die Dehydratisierung unsymmetrisch substituierter Polyalkylpiperidin-4-ole (II) führt naturgemäß zu Gemischen von Polyalkylpiperideinen (IIIa) und (IIIb). In diesem Fall werden bei der weiteren Umsetzung natürlich ebenfalls Gemische der Polyalkylpiperidindiole (Ia) und (Ib) gebildet.

Zur Herstellung der Polyalkylpiperidindiole kann man sich als Sauerstoffüberträger sowohl fertiger Persäuren als auch in situ erzeugter Persäuren bedienen. Man geht z. B. so vor, daß man das Polyalkylpiperidein (III) in einem inerten organischen Lösungsmittel wie Ether, Dichlorethan, Essigsäure oder bevorzugt Ameisensäure vorlegt und die äquimolare bis 1,2fach molare Menge, vorzugsweise die äquimolare Menge einer organischen Persäure wie Peressigsäure, Perbenzoesäure oder insbesondere Perameisensäure (in Form einer stabilen Lösung) bei 20 bis 40°C zutropft, worauf man 2 bis 20 Stunden nachrührt und ein Reaktionsgemisch erhält, welches die Epoxide bzw. — bei Einsatz von Perameisensäure — die Hydroxyformiate der Epoxide enthält.

Will man mit in situ erzeugten Persäuren arbeiten, so wird das Polyalkylpiperidein bei 20 bis 70, vorzugsweise bei 20 bis 50°C, zu einem Gemisch aus der 2- bis 20fachen, vorzugsweise der 2- bis 10fachen molaren Menge der als Sauerstoffüberträger vorgesehenen organischen Säure — insbesondere Ameisensäure oder auch Essigsäure — und der äquimolaren bis 1,2fach molaren, vorzugsweise 25- bis 60gew.-%igen Wasserstoffperoxids, bezogen auf zu epoxidierendes Polyalkylpiperidein (III) zugetropft, bis zur vollständigen Umsetzung 1 bis 20 Stunden bei ca. 20 bis 50°C nachgerührt und gegebenenfalls zur Zerstörung überschüssiger Peroxide 1 bis 2 Stunden auf 50 bis 60°C erhitzt.

Aus dem auf die eine oder andere Weise erhaltenen Reaktionsgemisch entfernt man im Vakuum die Hauptmenge des Lösungsmittels, alkalisiert in der Kälte mit einer wäßrigen Alkalilauge und erwärmt sodann 2 bis 20 Stunden auf 60 bis 100°C, um den Epoxiring unter Wasseranlagerung zu spalten und gegebenenfalls entstandene Ester zu verseifen. In der Regel bildet sich hierbei eine ölartige Schicht, die beim Abkühlen erstarrt und aus den gewünschten Diolen besteht. Die ausgeschiedenen Kristalle werden abfiltriert und durch Umkristallisieren, z. B. aus einem flüssigen Kohlenwasserstoff wie Toluol oder Hexan, gereinigt.

Die Herstellung von Polyalkylpiperidindiolen mit $R^1$ = Alkyl kann auch in der Weise erfolgen, daß man Produkte mit $R^1 = H$ nach bekannten Methoden nachträglich alkyliert; die N-Oxide sind aus den Verbindungen mit $R^1 = H$ durch Oxidation z. B. mit $H_2O_2$ in Gegenwart von Wolframat synthetisierbar.

Es war überraschend und nicht zu erwarten, daß die bei aliphatischen und cycloaliphatischen Olefinen an sich bekannte Epoxidierung mit nachfolgender Wasseranlagerung zu den Diolen

(Houben—Weyl, Bd. VI/3, S. 371—487 [1965]; Org. Reactions, Bd. 7, S. 378—433 [1953]) sich im vorliegenden Falle erfolgreich durchführen läßt, da mit der Bildung unerwünschter Nebenprodukte gerechnet werden mußte. Die als Ausgangsmaterialien für die Synthese der neuen Polyalkylpiperidindiole (I) eingesetzten Polyalkylpiperideine (III) besitzen bekanntlich sowohl die Olefin- als auch die Aminostruktur, und es war daher eine Konkurrenzreaktion (besonders bei $R^1 = H$) am Stickstoffatom der Piperideine, nämlich die Bildung von N-Oxiden entsprechend der bekannten Oxidierung von Aminen zu N-Oxiden, nicht auszuschließen (E. G. Rozantsev und V. D. Sholle, Synthesis, 1971, S. 190—202 und 401—414). Ein einheitlicher Verlauf der Reaktion wäre lediglich dann als möglich anzusehen gewesen, wenn von vornherein Verbindungen der Formel (II) mit $R^1 = O$ eingesetzt werden. Daß Polyalkylpiperideine (III) mit Persäuren, insbesondere Perameisensäure, auch wenn $R^1$ = Wasserstoff ist, nahezu ausschließlich in die Epoxide und nicht in die N-Oxide überführbar sind, ließ sich somit keineswegs vorhersehen.

Die neuen Polyalkylpiperidindiole sind als Lichtschutzmittel für synthetische Polymere geeignet, sie finden aufgrund ihrer Bifunktionalität jedoch in erster Linie als Ausgangsmaterialien bei der Synthese hochwertiger polymerer Stabilisatoren für Kunststoffe Verwendung. Sie unterscheiden sich damit vorteilhaft von den in den DE-A-2 126 954 und 2 204 659 beschriebenen, lediglich in 4-Stellung eine alkoholische OH-Gruppe tragenden Polyalkylpiperidinverbindungen, die — ebenso wie ihre Derivate — als Mittel gegen das Ausbleichen von Farbphotographien bzw. ebenfalls zum Stabilisieren von Polymeren vorgeschlagen worden sind, denen jedoch für den letztgenannten Zweck aufgrund der auf diesem Sektor laufend gestiegenen Anforderungen keine besondere Bedeutung zukommt, da sie wegen ihres niedrigen Molgewichts eine relativ hohe Flüchtigkeit und eine nicht ausreichende Auswaschresistenz besitzen.

Die folgenden Beispiele dienen der weiteren Erläuterung der Erfindung.

## Beispiel 1

### 2,2,6,6-Tetramethylpiperidin-3,4-diol

In eine Lösung aus 200 g 98- bis 100%iger Ameisensäure und 30 g 30%igem $H_2O_2$ werden innerhalb von 1,5 Stunden 38 g 2,2,6,6-Tetramethyl-3-piperidein getropft. Im Verlauf der Zugabe steigt die Temperatur auf ca. 65°C und wird durch Außenkühlung bei 60 bis 65°C gehalten. Nach dem Abklingen der Hauptreaktion wird 16 Stunden nachgerührt, sodann 2 Stunden auf 60°C erhitzt und hierauf im Vakuum auf ca. 80 ml eingeengt. Nun wird unter Kühlen mit ca. 200 ml 25%iger NaOH alkalisiert, 16 Stunden bei 80°C gerührt und die nach dem Abkühlen sich absetzende kristallisierende Schicht abgenutscht. Man erhält 20 g des gewünschten Diols in Form des Hydrats vom Fp. 105 bis 107°C. Durch Umkristallisation aus Toluol wird die freie Base vom Fp. 139 bis 140°C gewonnen. Das Hydrochlorid vom Fp. 252°C kann man darstellen, indem man die freie Base z. B. in Ether löst und Chlorwasserstoffgas einleitet.

## Beispiel 2

Die Verbindung des Beispiels 1 kann auch in folgender Weise erhalten werden:

13,9 g (0,1 Mol) 2,2,6,6-Tetramethyl-3-piperidein werden in 100 ml 98- bis 100%iger Ameisensäure und 10 g Wasser vorgelegt. Dazu tropft man 19 g 40%ige Peressigsäure, heizt vorsichtig auf 50 bis 55°C und rührt bei dieser Temperatur 24 Stunden. Anschließend wird wie in Beispiel 1 aufgearbeitet (vor dem Einengen muß eine Peroxidprobe negativ sein).

Es fallen 10 g weiße Kristalle an, die, aus Toluol umkristallisiert, bei 138 bis 140°C schmelzen.

## Beispiel 3

### 1-Methyl-2,2,6,6-tetramethylpiperidin-3,4-diol

In eine Lösung aus 230 g 98- bis 100%ige Ameisensäure und 50 g 30%igem $H_2O_2$ werden innerhalb von 1,5 Stunden 50 g 1-Methyl-2,2,6,6-tetramethyl-3-piperidein getropft. Die Temperatur steigt hierbei auf 65°C und wird durch Kühlung bei ca. 60 bis 65°C gehalten. Anschließend wird wie in Beispiel 1 angegeben verfahren. Das sich abscheidende Öl wird mit Petrolether überschichtet, wobei das gewünschte Diol allmählich auskristallisiert.

Es fallen 15 g Kristallisat an, die aus Hexan umkristallisiert einen Fp. von 78°C besitzen.

Beispiel 4

Die Verbindung nach Beispiel 3 kann auch in folgender Weise hergestellt werden:
3 g des nach Beispiel 1 oder 2 erhaltenen 2,2,6,6-Tetramethyl-piperidin-3,4-diols werden mit 10 g 98- bis 100%iger Ameisensäure und 10 g 30%igem Formaldehyd versetzt. Es wird 10 Stunden auf ca. 100° C erhitzt und sodann auf ca. 7 ml im Vakuum eingeengt. Der Rückstand wird mit 20 ml 25%iger NaOH alkalisiert, wobei sich ein Öl abscheidet. Nach dem Überschichten mit 20 ml Petroläther fallen Kristalle an, die aus Hexan umkristallisiert werden. 27,9 g, Fp. 78° C.

## Patentansprüche

1. Polyalkylpiperidindiole der allgemeinen Formel

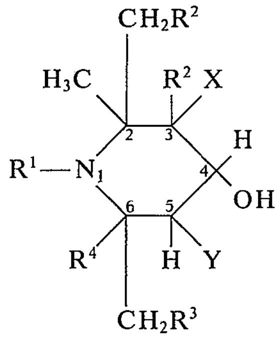

(I)

in der

X und Y verschieden sind und die Bedeutung eines Wasserstoffatoms oder einer Hydroxylgruppe haben,
$R^1$ Wasserstoff, Sauerstoff oder $C_1$- bis $C_{12}$-Alkyl ist,
$R^2$ und $R^3$ entweder gleich sind und Wasserstoff oder eine $C_1$- bis $C_5$-Alkylgruppe bedeuten, wobei dann
$R^4$ eine Methylgruppe ist, oder auch
$R^2$ Wasserstoff oder $C_1$- bis $C_5$-Alkyl ist und
$R^3$ und $R^4$ zusammen mit den Kohlenstoffatomen, an die sie gebunden sind, eine $C_5$- bis $C_6$-Cycloalkylgruppe oder eine Gruppe der Formel

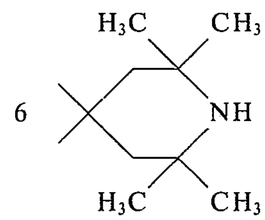

darstellen.

2. Verbindungen nach Anspruch 1, in denen $R^1$ = Wasserstoff oder Methyl, $R^2$ und $R^3$ Wasserstoff und $R^4$ Methyl ist.

3. Verfahren zur Herstellung der Polyalkylpiperidindiole nach den Ansprüchen 1 und 2, dadurch gekennzeichnet, daß man eine Verbindung der Formel (IIIa oder IIIb),

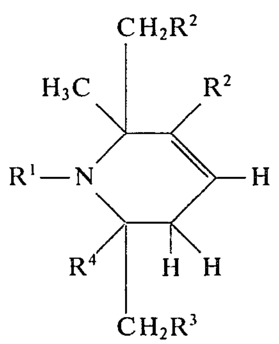

(III a)

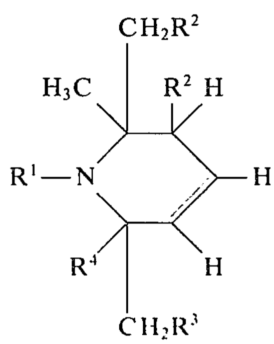

(III b)

in der $R^1$ bis $R^4$ die in Anspruch 1 angegebene Bedeutung haben, in Gegenwart eines organischen Lösungsmittels bei Temperaturen von 20 bis 70°C mit einer organischen Persäure, oder mit einer als Sauerstoff-Überträger fungierenden organischen Säure und $H_2O_2$ epoxidiert und hierauf die entstandenen Epoxide bzw. deren Säureadditionsprodukte durch Wasseranlagerung bzw. Verseifung in die gewünschten Diole überführt.

4. Verfahren nach Anspruch 3, dadurch gekennzeichnet, daß die Epoxidierung mit vorgebildeten oder mit in situ erzeugten Persäuren in Essigsäure und Ameisensäure erfolgt.

5. Verfahren nach Anspruch 3, dadurch gekennzeichnet, daß man mit vorgebildeter oder mit in situ erzeugter Perameisensäure in Ameisensäure als Lösungsmittel epoxidiert.

**Claims**

1. Polyalkyl-piperidine diols of the formula

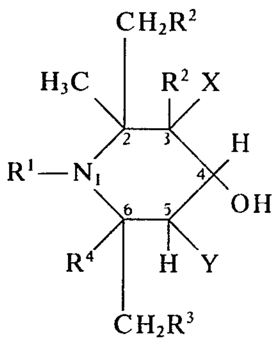

(I)

in which

X and Y are different and stand each for a hydroxyl group or a hydrogen atom;
$R^1$ is hydrogen, oxygen or $C_1-C_{12}$-alkyl,
$R^2$ and $R^3$ are either identical and stand each for hydrogen or a $C_1-C_5$ alkyl group,
$R^4$ being a methyl group in this case; or
$R^2$ is hydrogen or $C_1-C_5$-alkyl, and
$R^3$ and $R^4$ together with the carbon atoms to which they are linked form a $C_5$- or $C_6$-cycloalkyl group, or a group of the formula

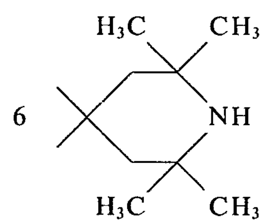

2. Compounds as claimed in Claim 1, in which $R^1$ is hydrogen or methyl, $R^2$ and $R^3$ are hydrogen, and $R^4$ is methyl.

3. A process for the preparation of the polyalkyl-piperidine diols as claimed in Claims 1 and 2, which comprises epoxydizing a compound of the formulae (IIIa) or (IIIb)

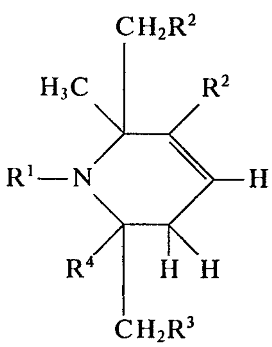

(III a)

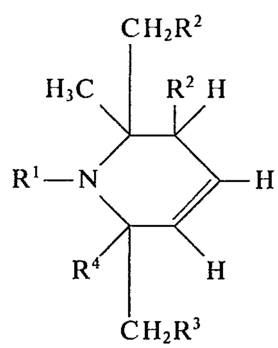

(III b)

in which $R^1$ through $R^4$ are as defined in Claim 1, in the presence of an organic solvent and at

temperatures of from 20 to 70°C with either an organic peracid, or an organic acid acting as oxygen carrier and $H_2O_2$, and subsequently converting the epoxides formed or their acid addition products to the intended diols by addition of water or by saponification.

4. The process as claimed in Claim 3, which comprises epoxidizing with the use of peracids, either preliminarily prepared or prepared in situ, in acetic or formic acid.

5. The process as claimed in Claim 3, which comprises epoxidizing with the use of performic acid, either preliminarily prepared or prepared in situ, in formic acid as solvent.

**Revendications**

1. Polyalkyl-pipéridine-diols répondant à la formule I

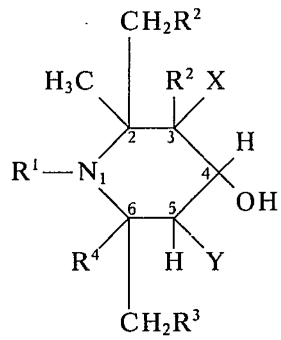

(I)

dans laquelle

X et Y sont différents l'un de l'autre et représentent chacun un groupe hydroxy ou un atome d'hydrogène,
$R^1$ représente l'hydrogène, l'oxygène, ou un radical alkyle en $C_1-C_{12}$, de préférence l'hydrogène ou un radical alkyle en $C_1-C_4$ et plus particulièrement l'hydrogène,
$R^2$ et $R^3$ sont soit identiques et ils représentent alors chacun l'hydrogène ou un radical alkyle en $C_1-C_5$, auquel cas
$R^4$ représente un radical méthyle, soit différents et alors;
$R^2$ représente l'hydrogène ou un radical alkyle en $C_1-C_5$ et
$R^3$ et $R^4$ forment ensemble et avec les atomes de carbone auxquels ils sont liés un radical cycloalkyle en $C_5$ ou $C_6$ ou un radical de formule

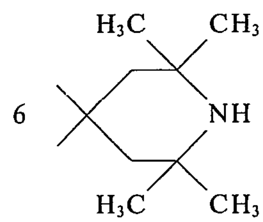

2. Composés selon la revendication 1, dans lesquels $R^1$ représente l'hydrogène ou un radical méthyle, $R^2$ et $R^3$ représentent chacun l'hydrogène et $R^4$ représente un radical méthyle.

3. Procédé de préparation de polyalkyl-pipéridine-diols selon l'une des revendications 1 et 2, procédé caractérisé en ce qu'on époxyde un composé répondant à l'une des formule IIIa et IIIb

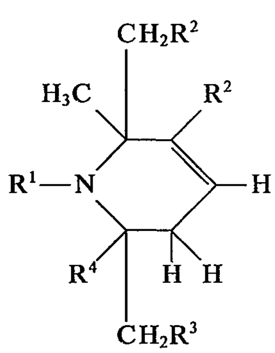

(III a)

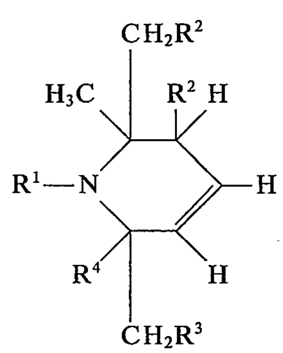

(III b)

dans lesquelles $R^1$ à $R^4$ ont les significations données à la revendication 1, en présence d'un solvant organique, à des températures de 20 à 70° C, avec un peracide organique, ou avec un acide organique fonctionnant comme un agent de transfert d'oxygène et $H_2O_2$, après quoi on transforme les époxydes formés, ou leur produits d'addition d'acides, par fixation d'eau ou par saponification, en les diols voulus.

4. Procédé selon la revendication 3, caractérisé en ce qu'on effectue l'époxydation avec des peracides préalablement formés ou créés in situ, dans l'acide acétique et l'acide formique.

5. Procédé selon la revendication 3, caractérisé en ce qu'on effectue l'époxydation avec de l'acide performique préalablement formé ou avec de l'acide performique créé in situ, dans l'acide formique comme solvant.